# EUROPEAN PATENT APPLICATION

(11) **EP 4 725 442 A1**
(43) Date of publication of application: **15.04.2026**
(21) Application number: 24205260.3
(22) Date of filing: 08.10.2024
(51) Int. Cl.: A61B 34/30, A61B 90/20, A61B 90/25, A61B 34/20, A61B 90/50

(54) **ROBOTIC IMAGING SYSTEM AND METHOD FOR ADAPTING A FOCUS POINT OF AN IMAGING DEVICE OF THE ROBOTIC IMAGING SYSTEM**

(71) Applicant: BHS Technologies GmbH, 6020 Innsbruck (AT)
(72) Inventor: BURGER, Gregor, 6176 Völs (AT); CAPEK, Carol, 6020 Innsbruck (AT); LEDERER, Lukas, 6020 Innsbruck (AT); PESCOLLER, Katharina, 6075 Tulfes (AT); MAIR, Michael, 6093 Rum (AT)
(74) Representative: Prüfer & Partner mbB Patentanwälte · Rechtsanwälte

(57) **Abstract**

The present invention relates to a robotic imaging system (100), comprising:
an imaging device (10) having at least one objective (11) associated with a focus point (11b) along an optical axis of the objective (11),
a robotic device (20) having at least one robotic arm (22, 23) connected to the imaging device (10) and comprising at least one pivot (23a, 24) and/or linear drive arranged between the imaging device (10) and the at least one robotic arm (22, 23), or the at least one robotic arm (22, 23) and another robotic arm (22, 23) or a robotic base (21) of the robotic device (20), wherein the at least one pivot (23a, 24) is configured to allow the imaging device (10) to be moved translationally and/or rotationally in at least one direction, and
a control device (30) configured to adapt the focus point (11b) of the imaging device (10), wherein
the at least one pivot (23a, 24) and/or linear drive comprises a detection unit (231a) configured to detect a position and/or angle of the at least one pivot (23a, 24) and/or linear drive, and/or a change in the position and/or angle of the at least one pivot (23a, 24) and/or linear drive, wherein
the detection unit (231a) is operatively connected to the control device (40) to transmit the detected position and/or angle, and or the detected change in the position and/or angle as a detection signal to the control device (30), and wherein,
in a manual mode in which the imaging device (10) is moved manually, the control device (30) is configured to adapt the focus point (11b) of the imaging device (10) in accordance with the transmitted detection signal such that the position of the focus point (11b) at the start of the manual movement is substantially the same as at the end of the manual movement.

## Description

The present invention relates to a robotic imaging system comprising an imaging device and a method for adapting a focus point of an imaging device of the robotic imaging system. The present invention further relates to a respective computer program product and a respective computer-readable storage medium allowing execution of the method.

For imaging, for example, by use of a robotic microscope system for medical procedures it is important to receive a sharp image of an object to be observed. Accordingly, the focus point of the imaging device of the robotic imaging system should always correspond to the region of interest with respect to the object to be observed. However, even if a focus point is appropriately set for one concrete pose of the imaging device with respect to the object to be observed, any change in the pose of the imaging device requires adaption of the focus point to keep the focus point in the same position as per the previous setting of the focus point before a respective change of the pose of the imaging system. Specifically with respect to a robotic imaging system allowing the imaging device being manually moved, keeping the focus in the same position is challenging since there such movement is not controlled by the robotic device in accordance with predetermined positioning data.

To keep the focus point in the same position after changing a pose of the imaging device, the imaging device may comprise an autofocus control based on detecting a marker on the object to be observed or any other kind of identification of the object to be observed. However, such approaches require respective sensors, particularly optical sensors, wherein an obstacle between the respective sensor and the object to be observed impedes an appropriate execution of the autofocus functionality. Further, respective sensors increase the complexity and costs of a robotic imaging system.

It is an object of the present invention to maintain the focus point in the same position when manually moving the imaging device.

The object is solved by the subject-matter of the independent claims. Further aspects of the present invention are subject to the dependent claims.

According to the present invention, a robotic imaging system comprises an imaging device having at least one objective associated with a focus point along an optical axis of the objective, and a robotic device having at least one robotic arm connected to the imaging device. The robotic device comprises at least one pivot and/or linear drive arranged between the imaging device and the at least one robotic arm, or the at least one robotic arm and another robotic arm or a robotic base of the robotic device. The at least one pivot and/or linear drive are/is configured to allow the imaging device to be moved translationally and/or rotationally in at least one direction. The robotic imaging system further comprises a control device configured to adapt the focus point of the imaging device. Further, the at least one pivot and/or linear drive comprises a detection unit configured to detect a position and/or angle of the at least one pivot and/or linear drive, and/or a change in the position and/or angle of the at least one pivot and/or linear drive. The detection unit is operatively connected to the control device to transmit the detected position and/or angle, and or the detected change in the position and/or angle as a detection signal to the control device. In a manual mode in which the imaging device is moved manually, the control device is configured to adapt the focus point of the imaging device in accordance with the transmitted detection signal such that the position of the focus point at the start of the manual movement is substantially the same as at the end of the manual movement.

In view of the above, a manual mode of the robotic imaging system is a mode in which a position and/or orientation of the imaging device can be manually changed by moving the imaging device directly or indirectly via the at least one robotic arm of the robotic device. The robotic device comprising the manual mode may be a so-called cobot. The robotic imaging system may also comprise a drive mode in which the position and/or orientation of the imaging system may be changed by controlling a respective drive of the robotic device by a control unit. Such control unit may be the control device to also adapt the focus point or comprised the control device or a separate control unit.

The robotic device comprising the at least one pivot and/or linear drive allows the imaging device to be moved in at least one degree of freedom. For example, the robotic device may comprise a first robotic arm with a first end connected to the imaging device via a pivot to allow a rotational movement of the imaging device relative to the first robotic arm. The second end of the first robotic arm is connected to a first end of a second robotic arm of the robotic device via another pivot to allow a rotational movement of the first robotic arm relative to the second robotic arm. Second end of the second robotic arm is connected to a robotic base via a further pivot to allow the second robotic arm to be rotationally moved relative to the robotic base. Alternatively or in addition to a respective pivot for a rotational movement, a linear drive may be implemented for a translational movement. The number of pivots and linear drives is not limited to a specific number as long as at least one degree of freedom for moving the imaging device is provided.

Accordingly, when the position and/or orientation of the imaging device is changed such change is based on a relative movement associated with the at least one pivot and/or linear drive. The detection unit detects the new positional state and/or a change in the positional state represented by an actual angle of the pivot or an actual position of the linear drive. A respective detection signal is transmitted to the control device to adapt the focus point accordingly to be positioned in substantially the same position as per the position of the focus point before the manual movement of the imaging device. The term "substantially" relates to minor deviations due to tolerances and the like.

The adaption of the focus point is based on knowing about movement made based on the detection signal, i.e. knowing about the new position and/or orientation of the imaging device. Specifically, for example, since the previous position of the focus point may be known as a reference position, the control device adapts the focus point as a focus point calculated from the current position of the imaging device to the reference position. Alternatively, the control device may adapt the focus point according to a deviation between the initial position and/or orientation of the imaging device and the current position of the imaging device as per the detection signal.

Since the robotic device may provide more than one pivot and/or linear drive each of which comprising its own detection unit, the control device may be also configured to receive any of the detection signals to determine the change in position and/or orientation of the imaging device.

In some embodiments, the detection unit is an encoder or comprises an encoder.

Encoders may already be implemented in the respective pivots and/or linear drives of the robotic device to be used in the drive mode to control the drive command and a respective execution of the commanded movement. Accordingly, in the manual mode, the same encoders can be used to control the adaption of the focus. A respective robotic device may thereby be retrofitted by adaption of the control to execute an adaption of the focus in the manual mode.

In some embodiments, the control device is configured to apply a conversion on the detected signal with respect to the adaption of the focus point.

For example, if the detected signal represents a tilting of the imaging device the control device converts the tilting into a change in distance of imaging device with respect to the reference position.

In some embodiments, the robotic imaging system comprises a gripping portion, preferably a handle, comprised by or attached to the imaging device or the robotic device to manually change a position and/or orientation of the imaging device.

The gripping portion may be gripped by an operator to manually move the imaging device or the robotic device respectively to change a position and/or orientation of the imaging device. Specifically, the gripping portion may comprise a detector such as a capacitive sensor for detection gripping of the gripping portion and therefore detecting a manual mode. The detection of gripping the gripping portion may also be used to release the manual mode.

Alternatively or in addition, the manual mode may be detected and/or released by another input device such as a foot switch. As a further variant, the manual mode may be detected by the presence of detection signals without the presence of control signals for respective drives or the presence of a drive mode, respectively.

In some embodiments, the at least one pivot and/or linear drive comprises at least one locking mechanism to keep the pivot and/or linear drive in a stationary state, and the control device is configured to release the at least one locking mechanism upon determination of the manual mode.

For example, the robotic device may comprise brakes as locking mechanisms for each of the pivots and/or linear drives. Accordingly, if a respective brake is applied to a respective pivot or linear drive, the respective pivot or linear drive is in a stationary state. To avoid any unintentional movement of the imaging device, the respective brake is only released upon determination of the manual mode or initiation of the manual mode, respectively.

In some embodiments, the robotic imaging system comprises a user interface configured to receive an input of an operator for the control device to adapt the focus in the manual mode.

Accordingly, the focus is not continuously adapted but only after a respective command input by the operator. This may allow to reduce the number of unnecessary focus adaptions until a final position and/or orientation of the imaging device is reached.

In some embodiments, the at least one objective of the imaging device is movable along the optical axis of the objective and/or is tiltable, and wherein the control device is configured to control the movement along the optical axis and/or the tilting angle of the at least one objective to adapt the focus point.

Accordingly, the focus point is adapted by adapting the position and/or orientation of the at least one objective.

In a further aspect, the present invention relates to a method for adapting a focus point of an imaging device of a robotic imaging system as previously described. The method comprises the steps of:
determining a position of the focus point associated with a current positional state of the at least one pivot and/or linear drive of the robotic device as reference position of the focus point,
changing a positional state of the at least one pivot and/or linear drive of the robotic device by moving the imaging device manually,
determining the changed positional state of the at least one pivot and/or linear drive of the robotic device due to the manual movement, and
adapting the focus point based on the determined changed positional state such that the position of the focus point is the same as the reference position of the focus point.

Any features or functionalities relating to the robotic imaging system with respect to the method are also applicable to the method. In turn, any features or functionalities relating to the method described with respect to the robotic imaging system are also applicable to the robotic imaging system.

In a further aspect, the present invention relates to a computer program product comprising instructions which, when the program is executed by a computer, cause the computer to carry out the method as previously described.

The computer may be a control device comprised by or connected to the robotic imaging system. Accordingly, the control device may be the control device of the robotic imaging system.

In a further aspect, the present invention relates to a computer-readable storage medium comprising instructions which, when executed by a computer, cause the computer to carry out the method as previously described.

Further advantages, aspects and details of the invention are subject to the claims, the following description of exemplary embodiments applying the principles of the invention, and the respective exemplary drawings.
Fig. 1- is a schematic representation of a robotic imaging system according to an exemplary embodiment of the present invention;
Fig. 2 is a schematic representation of an exemplary imaging device of the imaging system according to Fig. 1; and
Fig. 3 is a schematic representation of a first robotic arm and a second robotic arm of a robotic device according to Fig. 1.

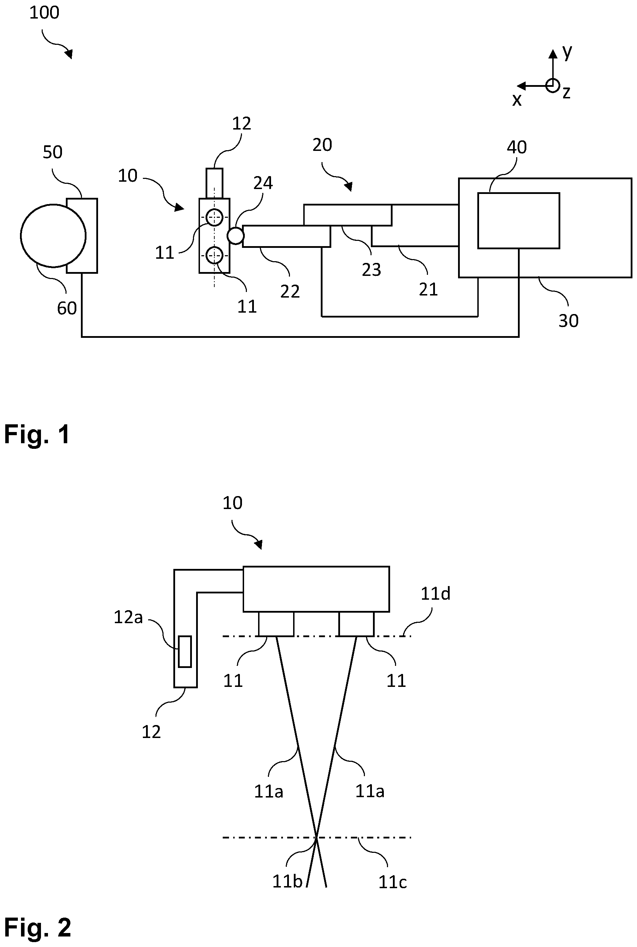

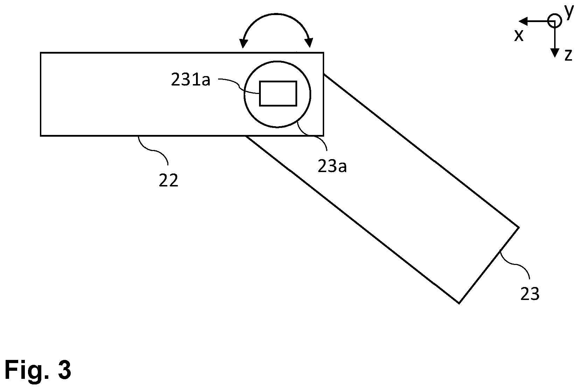

Fig. 1 shows a schematic representation of a robotic imaging system 100 according to an exemplary embodiment of the present invention. The imaging system 100 is a robotic microscope system comprising a stereoscopic imaging device as imaging device 10 with two objectives 11, a robotic device 20 to move and/or orient the imaging system 10, a control device 30 and a processing unit 40. In the exemplary embodiment, the control device 30 is comprised by the robotic device. However, in alternative embodiments, the control device 30 may be incorporated into the imaging device 10 or may be a separate device. The control device 30 is configured to control a movement and/or orienting of the imaging device 10 via the robotic device 20 in a drive mode. Specifically, the control device 30 is configured to control a position and/or orientation of the imaging device 10 by controlling a pivot 24 connecting the imaging device 10 to a first robotic arm 22, and by controlling a further pivot 23a (Fig. 3) connecting the first robotic arm 22 to a second robotic arm 23. The second robotic arm 23 is further connected stationary to a robotic base 21.

In a manual mode, the position and/or orientation of the imaging device 10 can be changed by the operator 60 gripping a handle as a gripping portion 12 attached to the imaging device 10 and moving the imaging device 10 in a desired position and/or orientation.

The control device 30 further comprises a processing unit 40 configured to process images received by the imaging device 10 and to transmit the processed images to a head-mounted display as display device 50 worn by an operator 60.

Further, Fig. 1 shows a cartesian coordinate system with a x-direction and y- direction extending within a horizontal plane and a z-direction being oriented in the direction of gravity.

Fig. 2 shows a schematic representation of the exemplary imaging device 10 of the imaging system 100 according to Fig. 1. Specifically, the representation provides a schematic front view of the imaging device 10 of the imaging system 100 according to the exemplary embodiment. Each of the two objectives 11 is directed to a common focus point 11b. In other words, the optical axes 11a of the objectives 11 intersect each other in their respective focus points represented by the common focus point 11b. A plane comprising the focus point 11b is a focal plane 11c. Further, the imaging device 10 comprises an objective plane 11d as averaged objective plane of the two objectives 11. The objective plane 11d corresponds to plane through the optical centers of the optical element or elements (not shown) of the objectives 11 with the optical axes 11a extending from such plane in direction of the focus point 11b.

Further, Fig. 2 shows the gripping portion 12 as a L-shaped handle with the free end to be gripped by the operator 60 comprising a capacitive sensor as detector 12a to detect gripping of the gripping portion 12. A signal to determine the gripping of gripping portion 12 is transmitted from the detector 12a to the control device 30. If the signal is representative of gripping the gripping portion 12, the control device determines a manual mode.

Fig. 3 shows a schematic representation of the first robotic arm 22 and the second robotic arm 23 of the robotic device 20 according to Fig. 1. The first robotic arm 22 is connected to the second robotic arm 23 via the pivot 23a to allow the first robotic arm to be moved rotationally relatively to the second robotic arm 23 about a respective rotation axis of the pivot 23a, here a rotational axis extending in the y-direction, as indicated by the double arrow. The relative position of the first robotic arm 22 with respect to the second robotic arm corresponds to a respective angle of the pivot 23a. The pivot 23a further comprises an encoder as detection unit 231a to detect the actual angle of the pivot 23a. A respective detection signal is transmitted to the control unit 30.

At the beginning of a rotational movement of the first robotic arm 22 with respect to the second robotic arm 23, the focus point 11b is in a reference position corresponding to a position of the focus point 11b being appropriate with respect to an object to be observed. In other words, the reference position of the focus point 11b is assumed as the desired position of the focus point 11b. Accordingly, the detection signal by the detection unit 231a represents a reference angle. The reference position and the reference angle are stored in the control device 30.

Upon moving the first robotic arm 22 with respect to the second robotic arm and therefore the imaging device 10 accordingly in the manual mode, the detection unit 231a detects a respective current angle of the pivot 23a. The corresponding detection signal is transferred to the control device 30 and is compared to the reference angle to calculate a current position and orientation of the imaging device 10. However, in alternative embodiments, the current position and orientation of the imaging device 10 is directly derived from the actual angle without comparison with the reference angle as an absolute position instead of a change in position. Since the control device has stored the reference position and has calculated the current position of the imaging device, the control device determines an angle and distance for the focus point 11b remaining in the reference position and adapts the objectives 11 accordingly.

The invention has been described with respect to exemplary embodiments. However, the invention is not limited to the exemplary embodiments. For example, the number of pivots and/or robotic arms may be different from the respective numbers provided by the exemplary embodiments. Further, the robotic imaging device may comprise more than one imaging device. The imaging device may also comprise only one objective. In another variant, the robotic device may be provided by at least one stand with at least one pivot or at least one linear guidance for changing a position of the imaging system in at least one degree of freedom with a respective detection unit to detect a respective change in a position and/or angle of the imaging system.

### LIST OF REFERENCE SIGNS

- 10: imaging device
- 11: objective
- 11a: optical axis
- 11b: focus point
- 11c: focus plane
- 11d: object plane
- 12: gripping portion
- 12a: detector
- 20: robotic device
- 21: robotic base
- 22: first robotic arm
- 23: second robotic arm
- 23a: pivot
- 24: pivot
- 30: control device
- 40: processing unit
- 50: display device
- 60: operator
- 100: robotic imaging system
- 231a: detection unit
- x: x-direction
- y: y-direction
- z: z-direction

## Claims

1. Robotic imaging system (100), comprising:
an imaging device (10) having at least one objective (11) associated with a focus point (11b) along an optical axis of the objective (11),
a robotic device (20) having at least one robotic arm (22, 23) connected to the imaging device (10) and comprising at least one pivot (23a, 24) and/or linear drive arranged between the imaging device (10) and the at least one robotic arm (22, 23), or the at least one robotic arm (22, 23) and another robotic arm (22, 23) or a robotic base (21) of the robotic device (20), wherein the at least one pivot (23a, 24) and/or linear drive are/is configured to allow the imaging device (10) to be moved translationally and/or rotationally in at least one direction, and
a control device (30) configured to adapt the focus point (11b) of the imaging device (10), wherein
the at least one pivot (23a, 24) and/or linear drive comprises a detection unit (231a) configured to detect a position and/or angle of the at least one pivot (23a, 24) and/or linear drive, and/or a change in the position and/or angle of the at least one pivot (23a, 24) and/or linear drive, wherein
the detection unit (231a) is operatively connected to the control device (40) to transmit the detected position and/or angle, and or the detected change in the position and/or angle as a detection signal to the control device (30), and wherein,
in a manual mode in which the imaging device (10) is moved manually, the control device (30) is configured to adapt the focus point (11b) of the imaging device (10) in accordance with the transmitted detection signal such that the position of the focus point (11b) at the start of the manual movement is substantially the same as at the end of the manual movement.

2. Robotic imaging system (100) according to claim 1, wherein the detection unit (231a) is an encoder or comprises an encoder.

3. Robotic imaging system (100) according to claim 1 or 2, wherein the control device (30) is configured to apply a conversion on the detected signal with respect to the adaption of the focus point (11b).

4. Robotic imaging system (100) according to any one of the preceding claims, wherein the robotic imaging system (100) comprises a gripping portion (12), preferably a handle, comprised by or attached to the imaging device (10) or the robotic device (20) to manually change a position and/or orientation of the imaging device (10).

5. Robotic imaging system (100) according to any one of the preceding claims, wherein the at least one pivot (23a, 24) and/or linear drive comprises at least one locking mechanism to keep the pivot (23a, 24) and/or linear drive in a stationary state, and the control device (30) is configured to release the at least one locking mechanism upon determination of the manual mode.

6. Robotic imaging system (100) according to any one of the preceding claims, wherein the robotic imaging system (100) comprises a user interface configured to receive an input of an operator (60) for the control device to adapt the focus in the manual mode.

7. Robotic imaging system (100) according to any one of the preceding claims, wherein the at least one objective (11) of the imaging device (10) is movable along the optical axis (11a) of the objective (11) and/or is tiltable, and wherein the control device (30) is configured to control the movement along the optical axis (11a) and/or the tilting angle of the at least one objective (11) to adapt the focus point.

8. Method for adapting a focus point (11b) of an imaging device (10) of a robotic imaging system (100) according to any one of the preceding claims, comprising the steps of:
determining a position of the focus point (11b) associated with a current positional state of the at least one pivot (23a, 24) and/or linear drive of the robotic device (20) as reference position of the focus point (11b),
changing a positional state of the at least one pivot (23a, 24) and/or linear drive of the robotic device (20) by moving the imaging device (10) manually,
determining the changed positional state of the at least one pivot (23a, 24) and/or linear drive of the robotic device (20) due to the manual movement, and
adapting the focus point (11b) based on the determined changed positional state such that the position of the focus point (11b) is the same as the reference position of the focus point (11b).

9. Computer program product comprising instructions which, when the program is executed by a computer, cause the computer to carry out the method according to claim 8.

10. Computer-readable storage medium comprising instructions which, when executed by a computer, cause the computer to carry out the method according to claim 8.
